# EUROPÄISCHE PATENTSCHRIFT

(11) **EP 1 684 146 B1**
(45) Veröffentlichungstag und Bekanntmachung des Hinweises auf die Patenterteilung: **30.07.2014**
(21) Anmeldenummer: 06000434.8
(22) Anmeldetag: 10.01.2006
(51) Int. Cl.: G05D 16/20, A61M 1/00, A61B 5/00

(54) **Verfahren zum Betrieb eines medizintechnischen Therapiegerätes**
Method of operating a medical therapy apparatus
Procédé d'utilisation d'un appareil médical thérapeutique

(30) Priorität: 14.01.2005 DE 102005001868
(43) Veröffentlichungstag der Anmeldung: 26.07.2006
(73) Patentinhaber: Paul Hartmann AG, 89522 Heidenheim (DE)
(72) Erfinder: Dirk Baumann, 20459 Hamburg (DE); Johannes Meyer, 20259 Hamburg (DE)

(56) Entgegenhaltungen:
- EP-A- 0 761 160
- EP-A- 1 356 762
- WO-A-96/05873
- CA-A1- 2 333 255
- DE-T2- 69 505 545
- FR-A- 2 717 332
- US-A- 5 553 609
- US-A1- 2002 143 290

## Beschreibung

Die vorliegende Erfindung betrifft ein Verfahren zum Betreiben eines mit einem elektronischen Steuergerät, mit mindestens einer elektrischen Pumpe, mindestens einem Ventil und mindestens einem Drucksensor versehenen medizinischen Therapiegerätes, insbesondere Anti-Dekubitus-Auflage und/oder System für Unterdruckbehandlung von Wunden, wobei Daten zwischen dem Steuergerät und einer Zentrale mittels bidirektionaler Datenübertragung ausgetauscht werden.

Die Erfindung betrifft gleichermaßen ein medizinisches Therapiegerät mit einem elektronischen Steuergerät, mindestens einer elektrischen Pumpe, mindestens einem Ventil und mindestens einem Drucksensor, insbesondere Anti-Dekubitus-Auflage und/oder System für Unterdruckbehandlung von Wunden, bei dem Daten zwischen dem Steuergerät mit einer seriellen Schnittstelle zur Verbindung mit dem Internet austauschbar sind.

Aus der WO 96/05873 A ist ein Therapiegerät mit einer Wunddrainagevorrichtung bekannt, bei dem ein elektronisches Steuergerät eine elektrische Vakuumpumpe steuert, die zur Erzeugung eines Unterdrucks in einer Wunde zur Förderung der Wundheilung dient.

Nachteilig an diesem vorbekannten Behandlungsgerät ist, dass eine Wartung des Gerätes stets erfordert, dass eine Wartungsperson das Gerät physisch zur Verfügung hat. Hieraus ergeben sich hohe Kosten durch An- und Abreise des Wartungspersonals und in der Praxis in der Regel auch verringerte Standzeiten des Therapiegerätes.

Aus der EP 1 356 762 A1 ist eine Geräteanordnung zur Fernüberwachung von Körperfunktionen bekannt, bei der ein Therapiegerät bei einem Patienten angeordnet und mittels bidirektionaler Datenfernübertragung mit einem von einem Arzt bedienbarer Rechner verbunden ist. Körperfunktionen des Patienten werden gemessen und die Messwerte an den Arzt übermittelt, der seinerseits ferngesteuerte Therapiemaßnahmen auslösen kann, beispielsweise die Infusion eines Medikaments. Auch eine Fernwartung des Therapiegerätes durch einen Techniker ist in begrenztem Umfang mäglich, indem Funktionskontrollsignale an das Gerät geschickt werden.

Aus der CA 2 333 255 ist ein tragbares medizinisches Messsystem für die Überwachung von Patienten bekannt, bei dem Patientendaten beispielsweise durch Funk übertragen werden können. Um die Bewegungen des Patienten zu überwachen, ist das Messsystem mit GPS ausgestattet.

Aus der US 2002/0143290 ist ein medizinisches Gerät zur ferngesteuerten medizinischen Behandlung von Patienten bekannt, bei dem ein internes Computerprogramm beim Einschalten einer Pumpe eine Initialisierung und einem Pumpentest ausführt.

Aus der FR 2 717 332 ist ein medizinisches Überwachungssystem bekannt, bei dem die zu übertragenden Daten verschlüsselt werden können.

Aufgabe der vorliegenden Erfindung ist es, ein Verfahren zum Betreiben eines Therapiegerätes der eingangs genannten Art anzugeben, durch welches die Ausfallzeiten minimiert werden, sowie eine optimale, am medizinischen Fortschritt zeitnah partizipierende Patientenversorgung ermöglicht wird.

Diese Aufgabe wird gemäß der Erfindung dadurch gelöst, dass bei einem gattungsgemäßen Verfahren zu Zwecken einer statistischen Auswertung Grunddaten und Betriebsdaten des Therapiegerätes vom Steuergerät an die Zentrale übertragen werden. Die erfindungsgemäße Maßnahme ermöglicht die Überwachung des ordnungsgemäßen Betriebs des Therapiegerätes. Insbesondere kann die Dichtigkeit einer Vakuumauflage bei einem Vakuumtherapiesystem überprüft werden oder auch die Dichtigkeit einer Anti-Dekubitus-Auflage, ohne dass Personal vor Ort am Einsatzort dieser Geräte präsent sein muss- Gegebenenfalls ist eine Fernwartung möglich.

Auf der anderen Seite können Daten zahlreicher an verschiedenen Orten aufgestellter Geräte an die Zentrale übermittelt werden, wodurch mit Vorteil bei geeigneter Wahl der übertragenen Daten eine Auswertung eines großen Patientenkollektivs im Sinne einer klinischen Multicenterstudie durchgeführt werden kann. Aus den Ergebnissen einer Auswertung der Daten des Patientenpools können verbesserte Regelalgorithmen oder optimierte Parameter für bekannte Regelalgorithmen bestimmt werden.

Aufgrund der Möglichkeit des bidirektionalen Datenaustausches ist es zudem mit Vorteil möglich, diese verbesserten Algorithmen oder optimierten Regelparameter an eines oder mehrere Steuergeräte von der Zentrale per Datenfernübertragung zu übertragen. Hierdurch kann jeder Therapieplatz mit minimalem zeitlichem Verzug und sehr kostengünstig ständig aktuell in Bezug auf die medizinische Erkenntnis gehalten werden. Die gemäß der Erfindung an verschiedenen voneinander entfernten Orten aufgestellten Therapiegeräte werden also zu einem Netzwerk zusammengeschaltet.

Alternativ zur Übertragung via Internet kann die Datenübertragung gemäß der Erfindung auch über Telefondirektverbindung erfolgen mit dem Vorteil, dass ein unbefugter Datenzugriff im Vergleich zu der Übertragung via Internet noch deutlich erschwert wird. Dabei kann die Telefondirektverbindung sowohl über Festnetz, GSM, UMTS oder einer anderen bekannten Telefondirektverbindung erfolgen.

Diese Flexibilität bei der konkreten Technologie für die Datenfernübertragung ermöglicht den Datenaustausch mit Steuergeräten nicht nur von Therapiegeräten, welche in modern ausgestatten Krankenhauseinrichtung betrieben werden, sondern auch von solchen Therapiegeräten, die beispielsweise beim Patienten zu Hause verwendet werden.

In Ausgestaltung des erfindungsgemäßen Verfahrens ist vorgesehen, dass die vom Steuergerät an die Zentrale übertragenen Daten mindestens eine der folgenden Grunddaten umfassen: Seriennummer des Steuergerätes; Steuergerätegrundeinstellungen, insbesondere Sprache und über Einheitensystem; Patientendaten, insbesondere Gewicht, Größe, Body-Mass-Index.

Durch die Übertragung der Seriennummer des Steuergerätes ist die Zuordnung der übertragenen Daten zu dem sendenden Gerät möglich, so dass bei einer Zusammenschaltung mehrerer Steuergeräte zu einem Netzwerk gemäß der oben beschriebenen Variante der Erfindung keine Verwechslungen auftreten.

Zur Interpretierung der übersendeten Messgrößen bzw. auch Fehlermeldungen ist es unerlässlich, die Grundeinstellungen des Steuergerätes zu kennen. Eine Auswertung von Messwerten kann sinnvoll naturgemäß nur erfolgen, wenn die verwendeten Einheiten dazu bekannt sind.

Die Kenntnis der Steuergerätegrundeinstellungen eines an einem entfernten Ort aufgestellten medizinischen Therapiegerätes ermöglicht gemäß der Erfindung mit Vorteil, dass auch eine Online-Hilfe bei Rückfragen des Bedieners oder des Patienten selber möglich ist.

Durch die Übersendung der Seriennummer des Steuergerätes als Identifizierungsmerkmal kann beim Einsatz von mehreren Systemen dezentral eine Datenanzeige, etwa im Schwesternzimmer inkl. der vom Gerät generierten Alarme erfolgen. Dies ist besonders in großen Krankenhausstationen von Vorteil, wo die Personalknappheit häufig dazu führt, dass medizinisches Personal nicht ständig an jedem Therapiegerät vor Ort anwesend sein kann.

Die Übermittlung von Gewicht, Größe und dem aus Gewicht und Größe berechenbaren Body-Mass-Index ist insbesondere zur statistischen Auswertung der Druck- und sonstigen Therapieparameter bei Anti-Dekubitus-Auflagen von Bedeutung. Die Auswertung in der Zentrale dieser Daten von einer Vielzahl von elektronischen Steuergeräten für Anti-Dekubitus-Auflagen oder Vakuumtherapiesystemen ermöglicht es, Regelalgorithmen bzw. Parameter für solche Algorithmen für bestimmte nach Gewicht, Größe bzw. Body-Mass-Index bestimmte Patientengruppen zu optimieren.

Eine klinische Multicenterstudie, welche Daten von den Anti-Dekubitus-Auflagen oder von Vakuumtherapiesystemen auswertet, lässt sich so vorteilhaft mit minimalem zusätzlichen Aufwand unter Einbeziehung von im Feld befindlichen Therapiegeräten, z.B. auch von Geräten, die bei Patienten zu Hause im Einsatz sind, durchführen. Eine schnelle und ständige Weiterentwicklung dieser Systeme ist daher mit Vorteil durch die ständige Auswertung von unmittelbar aus der Praxis gewonnenen Daten möglich.

Bei einer anderen speziellen Ausführungsform des erfindungsgemäßen Verfahrens umfassen die vom Steuergerät an die Zentrale übertragenen Daten mindestens eine der folgenden Betriebsdaten: Betriebslaufzeit; Pumpenlaufzeit; Anzahl der Ventilschaltungen; Soll- und Ist-Druckwert; Versorgungsspannung des Steuergerätes; Betriebszustand der Pumpe. Die Übermittlung der Betriebslaufzeit an die Zentrale kann mit Vorteil z. B. dazu verwendet werden, festzustellen, ob eine nach einer bestimmten Betriebslaufzeit vorgesehene Wartung zu erfolgen hat. Aus der Anzahl der Veritilschaltungen oder der Pumpenlaufzeit und der Betriebszeit kann mit Vorteil in der Zentrale berechnet werden, wie oft jedes einzelne Ventil im Durchschnitt pro Stunde geschaltet hat.

Außerdem kann ermittelt werden, wie lange die Pumpe gearbeitet hat. Durch Vergleich mit Erfahrungswerten lässt sich hieraus beispielsweise im Falle einer Anti-Dekubitus-Auflage erkennen, ob sich in der Auflage eine Leckage befindet.

Durch Fernabfrage von Soll- und Ist-Druckwerten und Fernübertragung gemäß der Erfindung kann beispielsweise in der Zentrale vom medizinischen Personal festgestellt werden, ob diese Werte für den Patienten geeignet sind. Dies ist insbesondere im Falle von einer Heimanwendung der Therapiegeräte vom großen Vorteil, wo wirksam verhindert werden muss, dass ein Patient etwa durch einen Bedienfehler das Gerät auf für ihn suboptimale Werte justiert.

Die Kenntnis der Versorgungsspannung des Steuergerätes erlaubt es, die Ursache für mögliches Fehlverhalten des Gerätes, welches häufig durch zu stark schwankende Versorgungsspannung im Falle des Netzbetriebes oder durch abnehmende Batterie- bzw. Akkumulatorspannung bedingt sein kann, zu ermitteln.

Die Übermittlung des Betriebszustandes der Pumpe gibt Aufschluss darüber, ob die Pumpe gerade läuft oder in Ruhestellung ist. Diese Information kann in Verbindung mit beispielsweise den Soll- und Ist-Druckwerten ebenfalls mit Vorteil zur Verifizierung eines ordnungsgemäßen Betriebs herangezogen werden.

In spezieller Ausgestaltung des erfindungsgemäßen Verfahrens sind die vom Steuergerät an die Zentrale übertragenen Daten mit einem Zeitstempel versehen. Die Auswertung der Daten wie z. B. Soll- und Ist-Druckwert etc. lassen sich auf diese Weise mit Vorteil auch in zeitlicher Abfolge auswerten. Hierdurch kann beispielsweise das erste Auftreten eines Fehlers mit physiologischen Veränderungen oder Veränderungen der Betriebsdaten zeitlich korreliert werden.

Wenn gemäß einer anderen Ausführungsform des erfindungsgemäßen Verfahrens die vom Steuergerät an die Zentrale übertragenen Daten von dem Steuergerät erzeugte Alarme umfassen, können diese in der Zentrale ausgewertet bzw. auch nur angezeigt werden. Die direkte Übermittlung der Alarme bzw. Fehlermeldungen kann gemäß der Erfindung mit Vorteil auch direkt an den Gerätehersteller oder den Wartungsvertragspartner erfolgen. Dies ermöglicht es den Herstellern bzw. den Wartungsvertragspartnern unverzüglich die erforderlichen Maßnahmen zu ergreifen. Daraus ergibt sich der Vorteil, dass besonders hohe Standzeiten bzw. geringe Ausfallzeiten der Geräte erreichbar sind.

Eine besonders zuverlässige Standortanalyse insbesondere von Therapiegeräten, welche beim Patienten zu Hause eingesetzt werden, ist möglich, wenn die vom Steuergerät an die Zentrale übertragenen Daten im Steuergerät mittels GPS (Global Positioning System) ermittelte Daten über einen momentanen Standort des Steuergerätes umfassen. Zweckmäßigerweise kann ein GPS-Empfänger zu diesem Zweck in das Therapiergerät integriert werden. Durch die Standortanalyse kann das in der Praxis häufig vorhandene Problem des Verlustes von zu Hause beim Patienten betriebenen Therapiegeräten mit Vorteil erheblich vermindert werden.

In anderer Ausgestaltung des erfindungsgemäßen Verfahrens umfassen die von der Zentrale an das Steuergerät übertragenen Daten mindestens eine der folgenden Daten: Software-Aktualisierungen; Patienten-Parameter, insbesondere Größe oder Gewicht; Regelparameter, insbesondere Solldruckwerte; Steuerbefehle, insbesondere Befehle zum Löschen oder das Zurücksetzen von auf dem Steuergerät gespeicherten Daten. Die Möglichkeit, Software-Aktualisierungen per Fernübertragung an das Steuergerät zu übertragen, dient wiederum mit Vorteil zur Verminderung der Wartungskosten.

Die Möglichkeit, Patienten-Parameter wie Größe und Gewicht von der Zentrale an das Steuergerät zu übertragen, ermöglicht es mit Vorteil, beispielsweise vor Ort fehlerhaft vorgenommene Eintragungen dieser Parameter durch Fernwartung zu korrigieren. Dies ist insbesondere bei der Verwendung des erfindungsgemäßen Verfahrens im Betrieb von beim Patienten zu Hause aufgestellten medizinischen Therapiegeräten, wie z. B. Anti-Dekubitus-Auflagen und/oder Systemen zur Unterdruckbehandlung von Wunden von großer Bedeutung. Außerdem können diese übertragenen Daten zu einer Standortanalyse herangezogen werden, um beispielsweise den Ort zu ermitteln, an dem sich ein Gerät befindet bzw. sich zum Zeitpunkt der letzten Datenübertragung befunden hat. Dies ist insbesondere für Gesundheitsträger wie zum Beispiel Krankenkassen von Vorteil, wenn die Geräte zu Hause beim Patienten eingesetzt werden, um zu verhindern, daß Geräte versehentlich oder aus anderen Gründen abhanden kommen.

Die Möglichkeit, von der Zentrale an das Steuergerät Regelparameter, besonders Solldruckwerte, zu übertragen, eröffnet die Möglichkeit, aus beispielsweise statistischen Auswertungen berechnete verbesserte Regelungsparameter zeitnah und mit minimalem Aufwand an das Steuergerät zu übertragen, so dass alle mit der Zentrale verbundenen Steuergeräte an diesem medizinischen Fortschritt teilhaben können.

Die Übermittlung von Steuerbefehlen von der Zentrale an das Steuergerät ermöglicht es mit Vorteil, den Ablauf der medizinischen Therapie aus der Ferne direkt zu beeinflussen. Etwa, wenn ein Patient, der beispielsweise eine Anti-Dekubitus-Auflage zu Hause verwendet, über plötzlich aufgetretene Beschwerden seit Beginn der Therapie fernmündlich klagt, kann daran gedacht werden, den Druck in der Auflage in geeigneter Weise direkt von der Zentrale aus zu verändern.

Die Option, auf dem Steuergerät gespeicherte Daten zu löschen oder zurückzusetzen, dient dazu, Daten, die zusätzlich oder ausschließlich zu der erfindungsgemäßen Datenfernübertragung lokal auf dem Steuergerät abgelegt sind, zu verwalten. Die Steuerbefehle können aber auch Einschaltbefehle an die Pumpe oder der Befehl zum Öffnen oder Schließen des Ventils sein.

Bei einer speziellen Weiterbildung des erfindungsgemäßen Verfahrens umfassen die von der Zentrale an das Steuergerät übertragenen Daten eine Befehlsfolge zur Durchführung eines Funktionstests an dem Therapiegerät. Hiermit kann mit Vorteil gezielt überprüft werden, ob sich das Gerät wie vorgesehen verhält. Je nach dem Ergebnis kann entschieden werden, ob und ggf. welche Wartung erforderlich ist.

Gemäß einer speziellen Variante des Verfahrens nach der Erfindung ist der Funktionstest eine Leckageprüfung des Therapiegerätes, welcher die folgenden Schritte umfasst:
- Bestimmen der Pumpenlaufzeit und der Betriebszeit während eines vorbestimmten Zeitintervalls und/oder
- Bestimmen der Anzahl der Ventilschaltungen während eines vorbestimmten Zeitintervalls
- Feststellen, ob eine Leckage vorliegt, durch Vergleich mit Erfahrungswerten.

Bei diesem erfindungsgemäßen Funktionstest können per Fernüberwachung sicherheitstechnische Kontrollen, die beispielsweise gemäß dem deutschen Medizinproduktegesetz (MPG) vorgeschrieben sind, sowie Service-Checks durchgeführt werden, ohne dass ein Techniker vor Ort zu sein braucht. Bei dem Test lassen sich mit Vorteil Ventile und Pumpe für eine einstellbare Zeit in verschiedenen Kombinationen schalten. Über den Druckverlauf dieser Zeit lassen sich Aussagen über den Durchfluss und die Dichtigkeit sowohl des Gerätes als auch der Auflage treffen.

Der erfindungsgemäße Funktionstest wird noch verbessert, wenn das Feststellen durch eine in der Zentrale implementierte Automatik erfolgt. Dies ist insbesondere für den Fall, daß eine größere Anzahl medizinischer Therapiegeräte mit der Zentrale Daten austauscht von großem Vorteil.

Die der vorliegende Erfindung zugrunde liegende Aufgabe wird gleichermaßen gelöst durch ein medizinisches Therapiesystem mit einem elektronischen Steuergerät, mindestens einer elektrischen, mindestens einem Ventil und mindestens einem Drucksensor, insbesondere Anti-Dekubitus-Auflage und/oder System zur Unterdruckbehandlung von Wunden, welches gemäß der Erfindung zu Zwecken einer statistischen Auswertung von Daten und/oder zur Fernwartung für einen Austausch von Daten zwischen dem Steuergerät und einer Zentrale mittels bidirektionaler Datenfernübertragen mit einer seriellen Schnittschnelle, vorzugsweise RS 232 und/oder USB -Schnittstelle, und/oder einer Blue Tooth-Schnittstelle und/oder einer W-LAN-Schnittstnelle zur Verbindung mit dem Internet über ein Modem versehen ist.

Mit dem erfindungsgemäß ausgestalteten medizinischen Therapiegerät lassen sich die oben beschriebenen erfindungsgemäßen Verfahren durchführen und somit die Vorteile, die oben erläutert wurden, erzielen. Insbesondere können die Ausfallzeiten des Gerätes besonders gering gehalten werden, die Wartung kann sehr kostengünstig erfolgen, was insgesamt zu sehr hohen Standzeiten führt. Ferner kann das Gerät problemlos und zeitnah an den medizinischen Fortschritt angepasst werden, in dem ständig aktualisierte und optimierte Regelparameter und ähnliches an das Gerät fernübertragen werden.

Der steigenden Sensibilität gegenüber unbefugtem Zugriff auf vertrauliche Daten, insbesondere die Gesundheit von Patienten betreffend, kann mit einer speziellen Ausgestaltung des medizinischen Therapiegerätes gemäß der vorliegenden Erfindung entsprochen werden, in dem es zur Verschlüsselung der zu übertragenden Daten ausgebildet ist.

Die Erfindung wird in einer bevorzugten Ausführungsform unter Bezugnahme auf eine Zeichnung beispielhaft beschrieben, wobei weitere vorteilhafte Einzelheiten den Figuren der Zeichnung zu entnehmen sind.

Funktionsmäßig gleiche Teile sind dabei mit denselben Bezugszeichen versehen.

Die Figuren der Zeichnung zeigen im Einzelnen:
- Fig. 1:: schematische Darstellung des Ablaufs des erfindungsgemäßen Verfahrens bei einem elektronischen Steuergerät
- Fig. 2:: schematische Darstellung des Ablaufs einer Variante des erfindungsgemäßen Verfahrens mit einem Netzwerk von mehreren Steuergeräten.

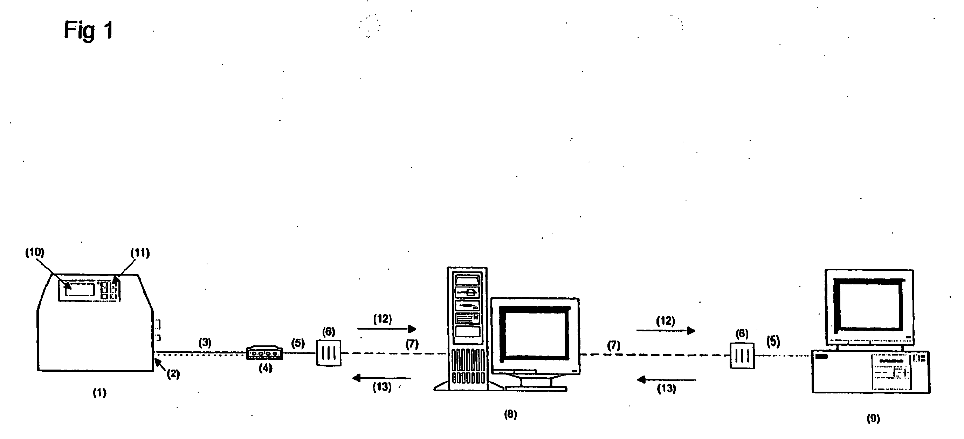

In der Fig. 1 ist ein elektronisches Steuergerät 1 zu erkennen, welches Bestandteil einer Anti-Dekubitus-Auflage mit einer elektrischen Pumpe, einem Ventil und mehreren Drucksensoren ist. Das elektronische Steuergerät 1 hat eine Schnittstelle 2. Diese ist wahlweise als RS 232-Schnittstelle, als USB-Schnittstelle, als Blue Tooth-Schnittstelle oder als W-LAN-Schnittstelle ausgestaltet. Über ein Schnittstellenkabel 3 ist die Schnittstelle 2 mit einem Modem 4 verbunden.

Das Modem 4 kann ein Analogmodem sein oder nach dem ISDN, DSL, GSM, UMTS oder GPRS Standard arbeiten. Der Ausgang des Modems 4 ist über ein Telefonkabel 5 mit einer Telefondose 6 verbunden. Die Telefondose ist in geeigneter Weise mit dem Internet 7 verbunden. An dem Internet 7 ist ein Kommunikationsserver 8 angeschlossen. Rechts in der Fig. 1 ist ein Datenserver 9 zu erkennen, welcher ebenfalls über ein Telefonkabel 5 an eine Telefondose 6 und hierüber an das Internet 7 angeschlossen ist.

Zur Übertragung von Daten von dem Steuergerät 1 an den Datenserver 9 werden zunächst Daten über die Schnittschnelle 2 des Steuergeräts 1 über das Schnittstellenkabel 3 an das Modem 4 übertragen. Die übertragenen Daten können Patientendaten sein, welche zuvor über die Tastatur eines lokalen Bedienfelds 10 vom medizinischen Personal oder auch vom Patienten direkt eingegeben worden sind.

Innerhalb des Modems 4 erfolgt eine Modulierung der Daten nach einem der genannten Protokolle. Anschließend werden wir die modulierten Daten über das Telefonkabel 5 und die Telefondose 6 und das Internet 7 an den Kommunikationsserver 8 übertragen.

Das Steuergerät 1, das Schnittstellenkabel 3, das Modem 4, das Telefonkabel 5 sowie die Telefondose 6 befinden sich am Einsatzort beim Patienten, also beispielsweise im Krankenhaus am Krankenbett oder auch zu Hause beim Patienten.

Die von dem Steuergerät 1 abzufragenden Daten werden über das Internet 7 von dem Kommunikationsserver 8 empfangen. Der Kommunikationsserver 8 befindet sich dabei entweder am Ort des Geräteherstellers oder beispielsweise in einem zentralen Patientenüberwachungsraum wie z. B. dem Schwesternzimmer. Insbesondere ist es nicht erforderlich, dass sich der Kommunikationsserver 8 am selben Ort wie das Steuergerät 1 befindet.

Der Kommunikationsserver 8 überträgt die vom Steuergerät 1 empfangenen Daten wiederum über das Internet 7 und die Telefondose 6 sowie das Telefonkabel 5 an den Datenserver 9.

Alternativ zu der hier beispielhaft dargestellten Übertragung der Daten über das Internet ist es im Rahmen der Erfindung gleichermaßen möglich, die Daten über eine direkte Telephonverbindung zwischen dem Steuergerät und der Zentrale auszutauschen oder jede andere dem Fachmann bekannte Form der Datenfernübertragung zu verwenden.

Die Daten umfassen neben den vom medizinischen Bedienpersonal oder Patienten über die Tastatur 11 am lokalen Bedienfeld 10 des Steuergeräts 1 eingegebenen Patientendaten auch die Seriennummer des Steuergerätes 1, die Spracheinstellung des Steuergeräts 1 und das am Steuergerät 1 ausgewählte Einheitensystem. Ferner werden die Betriebslaufzeit des Steuergerätes 1, die Pumpenlaufzeit, die Anzahl der Ventilschaltungen, der Soll- und Ist-Druckwert, die Versorgungsspanne des Steuergerätes sowie der momentane Betriebszustand der Pumpe (in Betrieb / außer Betrieb) angezeigt. Alle übertragenen Daten sind mit einem Zeitstempel versehen, damit jedem übermittelten Wert ein Zeitpunkt zugeordnet werden kann, zu dem der jeweilige Wert ermittelt wurde.

Diese vom Steuergerät 1 an den Datenserver 9 übertragenen Daten werden im Datenserver 9 in einer Datenbank abgespeichert. Die Abspeicherung erfolgt in der Art, dass ein Abruf der Daten nach Geräteseriennummer sowie nach Abfragekriterien bezüglich beliebiger übertragenen Daten möglich ist. Aus den Daten wird in dem Datenserver 9 einerseits eine Auswertung des Verlaufs der mit der Anti-Dekubitus-Auflage unter Vermittlung des Steuergerätes 1 verabreichten Therapie durchgeführt. Hieraus lassen sich wichtige Parameter bezüglich des weiteren medizinisch indizierten Therapieverlaufs entnehmen. Die Daten werden also für eine Auswertung der Daten des einzelnen Patienten herangezogen.

Auf der anderen Seite werden die auf dem Datenserver 9 abgelegten Daten - sowohl Patientendaten als auch Messdaten des Steuergeräts 1 - einer patientenübergreifenden Auswertung zugänglich gemacht. Zu diesem Zweck wird das erfindungsgemäße Verfahren zum Übertragen der Daten vom Steuergerät 1 auf den Datenserver 9 nacheinander für verschiedene Patienten durchgeführt.

Die Daten werden jeweils in der Datenbank auf dem Datenserver 9 abgelegt. Anschließend erfolgt eine statistische Auswertung der auf diese Weise von mehreren Patienten, welche an dem von dem Steuergerät 1 gesteuerten medizinischen Therapiegerät behandelt wurden, gewonnenen Daten durchgeführt. Die Patientendaten können aus Gründen des Datenschutzes anonymisiert in der Datenbank abgelegt werden.

Hieraus lassen sich durch verschiedene bekannte Auswertemethoden, beispielsweise durch die Verwendung von neuronalen Netzen für die Bildung von Patientengruppen ähnlichen Therapieverlaufs oder ähnlicher Patientenparameter, Erkenntnisse für die Optimierung des dem medizinischen Therapiegeräts zugrunde liegenden Therapieverfahrens gewinnen. Aufgrund der Auswertung werden die Regelparameter angepasst.

Bei dem beschriebenen Verfahren zur Datenübertragung vom Therapiegerät 1 zum Datenserver 9 verläuft der Informationsfluss in Richtung des Pfeiles 12.

Außerdem werden Daten von dem Datenserver 9 an das Steuergerät 1 übertragen. Die Informationsflussrichtung für diese Datenübertragung ist in der Fig. 1 mit dem Pfeil 13 veranschaulicht. Für den Datenübertrag von dem Datenserver 9 an das Steuergerät 1 werden die Daten über die Telefonleitung 5 und die Telefondose 6 in das Internet 7 eingespeist und von dort an den Kommunikationsserver 8 weitergeleitet.

Der Kommunikationsserver 8 überträgt sodann die zu übertragenden Daten auf das Internet 7, wobei der Informationsfluss entlang des Pfeiles 13 zu der Telefonsdose 6 und von dort aus über das Telefonkabel 5 an das Modem 4 verläuft.

Innerhalb des Modems 4 erfolgt nach einem der oben genannten Protokolle die Demodulierung des empfangenen Signals. Das demodulierte Signal wird anschließend über das Schnittstellenkabel 3 und die Schnittstelle 2 an das Steuergerät 1 übertragen.

Im ersten beispielhaften Fall gemäß der Erfindung betreffen die in der beschriebenen Weise übertragenen Daten Parametereinstellungen, welche sich aufgrund der oben beschriebenen Auswertung des patientenübergreifenden Datenbestandes geändert haben und welche dementsprechend auf dem Steuergerät 1 anzupassen sind.

Im zweiten beispielhaften Fall betreffen die auf die beschriebene Weise übertragenen Daten eine Leckageprüfung der Anti-Dekubitus-Auflage. Dazu werden nacheinander Befehle zum Bestimmen der Pumpenlaufzeit und der Betriebszeit während eines vorbestimmten Zeitintervalls sowie zum Bestimmen der Anzahl der Ventilschaltungen während eines vorbestimmten Zeitintervalls übertragen.

Diese Befehle veranlassen die Logik des Steuergerätes 1 dazu, die angeforderten Daten zu ermitteln und diese auf die oben beschriebene Weise an den Datenserver 9 zu leiten, entlang der Informationsflussrichtung 12. In dem Datenserver wird anschließend aus der Anzahl der Ventilschaltungen und der Pumpenlaufzeit und der Betriebszeit berechnet, wie oft jedes einzelne Ventil im Durchschnitt pro Stunde geschaltet hat und wie lange die Pumpe gearbeitet hat.

Anschließend wird ein Vergleich mit in dem Datenserver 9 abgelegten Erfahrungswerten ermittelt, ob eine Leckage in der Auflage vorhanden ist.

Auf diese Weise kann per Fernwartung eine Leckage auf kostengünstige Weise ermittelt werden, bevor die Leckage ein Ausmaß erreicht, welches den Therapieerfolg beeinträchtigen könnte.

In einem dritten Ausführungsbeispiel betreffen die auf das Steuergerät 1 zu übertragenden Daten den Befehl, die Ventile und Pumpe der Anti-Dekubitus-Auflage für eine vorbestimmte Zeit in verschiedenen Kombinationen zu schalten. Der Datenserver 9 fragt anschließend vom Steuergerät 1 die Druckdaten ab, welche vom Steuergerät 1 auf die beschriebene Weise entlang der Informationsflussrichtung 12 an den Datenserver 9 inklusive eines Zeitstempels übermittelt werden.

Anhand dieser Informationen wird im Datenserver 9 der Druckverlauf während des vorbestimmten Zeitintervalls ausgewertet und es werden Aussagen über den Durchfluss und die Dichtigkeit sowohl des Steuergerätes als auch der Anti-Dekubitus-Auflage getroffen.

Auf diese Weise können mit Vorteil sicherheitstechnische Kontrollen gemäß Medizinproduktegesetz u. ä. durchgeführt werden, ohne dass ein

Techniker vor Ort zu sein braucht.

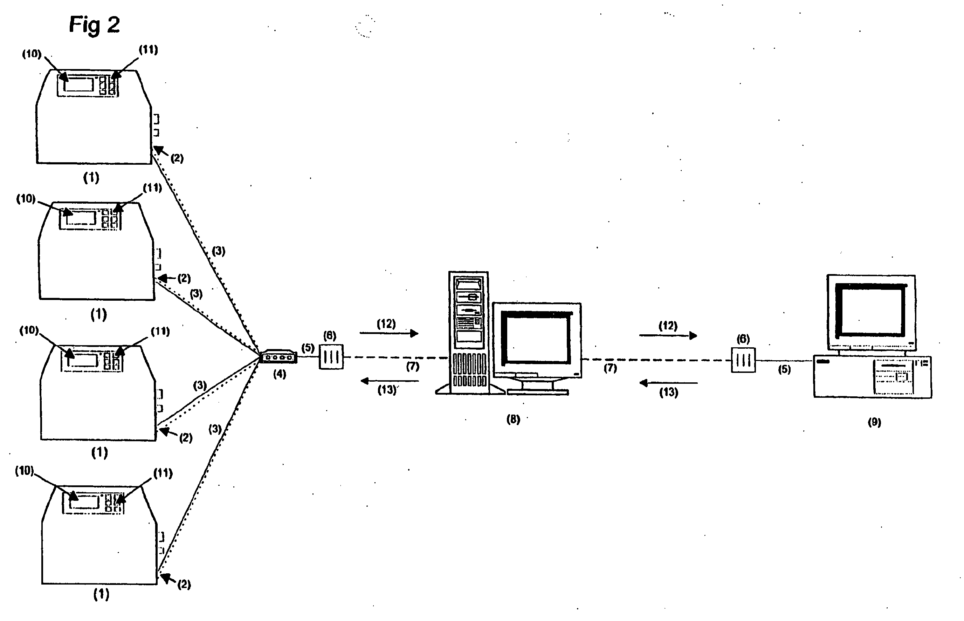

In der Fig. 2 ist eine Weiterbildung der Erfindung schematisch dargestellt, welche insbesondere für die Verwendung in Krankenhäusern oder Pflegeeinrichtungen geeignet ist. Dabei entspricht der Aufbau im wesentlichen den bereits in der Fig. 1 dargestellteri und dort näher erläuterten. Jedoch sind mit dem Modem 4 in diesem Falle beispielhaft insgesamt vier Steuergeräte 1 jeweils mit lokalen Bedienfeldern 10 und Tastaturen 11 verbunden. Die Verbindung zwischen dem jeweiligen Steuergerät 1 und dem Modem 4 ist wiederum durch die Schnittstelle 2 am Steuergerät 1 und das zwischen der Schnittstelle 2 und Modem 4 verlaufende Schnittstellenkabel 3 realisiert.

Die vier Steuergeräte - der Fachmann erkennt ohne weiteres, dass jede andere beliebige Zahl von Steuergeräten im Rahmen der Erfindung verwendet werden kann - sind somit über den Datenserver 9 zu einem Netzwerk zusammengeschaltet.

Die Datenabfrage entlang der Informationsflussrichtung 12, welche bekanntlich zur Datenübertragung vom Steuergerät 1 an den Datenserver 9 dient, erfolgt dabei in der oben beschriebenen Weise über die Schnittstelle 2, das Schnittstellenkabel 3, das Modem 4, das Telefonkabel 5, die Telefondose 6, das Internet 7, den Kommunikationsserver 8, das Internet 7, die Telefondose 6, das Telefonkabel 5 an den Datenserver 9.

Zur ebenfalls oben beschriebenen statistischen Auswertung werden gemäß dem in Fig. 2 dargestellten Netzwerk jedoch Daten aller an das Netzwerk angeschlossenen Steuergeräte 1 berücksichtigt, wobei mit jedem Steuergerät 1 nacheinander mehrere Patienten therapiert werden, was dazu führt, dass der Datenbank in dem Datenserver 9 in kurzer Zeit eine außerordentliche große Anzahl von Daten für eine statistische Auswertung zugespielt werden. Diese werden in dem Datenserver 9 in der oben beschriebenen Weise ausgewertet und zur Berechnung, Anpassung bzw. Optimierung von Regelalgorithmen oder Regelparametern verwendet.

Die ermittelten neuen Regelparameter werden in der oben beschriebenen Weise von dem Datenserver 9 an jedes an das Netzwerk angeschlossene Steuergerät 1 übertragen. Auf diese Weise werden mit großem Vorteil alle an das Netzwerk angeschlossene Steuergeräte 1 in kürzester Zeit mit minimalem Aufwand aufgrund der von einer großen Anzahl Patienten gewonnenen Daten aktualisiert.

Außerdem kann jedes der Steuergeräte 1, welche an das Netzwerk angeschlossen sind, Alarmmeldungen an den Datenserver 9 übermitteln, wo diese Alarmmeldungen anschließend ausgewertet werden und je nach Ergebnis der Auswertung die erforderlichen Wartungsmaßnahmen unverzüglich eingeleitet werden.

Mit einem solchen System können sich Standzeiten der Therapiesysteme im Krankenhaus oder zu Hause beim Patienten z. B. bis auf sieben Jahre verlängern lassen. Auch können Servicechecks inklusive elektronischer Signaturen zur Freigabe per Datenfernübertragung übermittelt werden. Ein Einschicken der Geräte zu einem Servicecheck entfällt auf diese Weise.

Der Fachmann erkennt, dass das anhand der Figuren beispielhaft beschriebene Verfahren im Rahmen der Erfindung ebenso beispielsweise auf Systeme zur Unterdruckbehandlung von Wunden oder andere Geräte angewendet werden kann.

Auf diese Weise ist mit dem erfindungsgemäßen Verfahren ein Verfahren angegeben, welches geringe Ausfallzeiten bei den medizinischen Therapiegeräten sowie hohe Standzeiten sicherstellt, eine kostengünstige Wartung ermöglicht und darüber hinaus eine optimale, dem medizinischen Fortschritt zeitnah angepasste Versorgung des Patienten sicherstellt.

### BEZUGSZEICHENLISTE

- 1: Steuergerät
- 2: Schnittstelle
- 3: Schnittstellenkabel
- 4: Modem
- 5: Telefonkabel
- 6: Telefondose
- 7: Internet
- 8: Kommunikationsserver
- 9: Datenserver
- 10: lokales Bedienfeld
- 11: Tastatur
- 12: Informationsflußrichtung Datenabfrage
- 13: Informationsflußrichtung Datenübertragung

## Patentansprüche

1. Verfahren zum Betreiben eines mit einem elektronischen Steuergerät (1), mindestens einer elektrischen Pumpe, mindestens einem Ventil und mindestens einem Drucksensor versehenen medizinischen Therapiegerätes, insbesondere Anti-Dekubitus-Auflage und/oder System zur Unterdruckbehandlung von Wunden, wobei Daten zwischen dem Steuergerät (1) und einer Zentrale (9) mittels bidirektionaler Datenfernübertragung ausgetauscht werden, **dadurch gekennzeichnet, dass** zu Zwecken einer statistischen Auswertung Grunddaten und Betriebsdaten des Therapiegerätes vom Steuergerät (1) an die Zentrale (9) übertragen werden.

2. Verfahren nach Anspruch 1, **dadurch gekennzeichnet, dass** die vom Steuergerät (1) an die Zentrale (9) übertragenen Daten mindestens eine der folgenden Grunddaten umfassen: Seriennummer des Steuergerätes (1); Steuergerätegrundeinstellungen, insbesondere Sprache und/oder Einheitensystem.

3. Verfahren nach Anspruch 1 oder 2, **dadurch gekennzeichnet, dass** die vom Steuergerät (1) an die Zentrale (9) übertragenen Daten mindestens eine der folgenden Betriebsdaten umfassen: Betriebslaufzeit; Pumpenlaufzeit; Anzahl der Ventilschaltungen; Soll- und Ist Druckwert; Versorgungsspannung des Steuergerätes (1); Betriebszustand der Pumpe.

4. Verfahren nach Anspruch 1 oder 2, **dadurch gekennzeichnet, dass** die vom Steuergerät (1) an die Zentrale (9) übertragenen Daten mit einem Zeitstempel versehen sind.

5. Verfahren nach einem der vorhergehenden Ansprüche, **dadurch gekennzeichnet, dass** die vom Steuergerät (1) an die Zentrale (9) übertragenen Daten von dem Steuergerät (1) erzeugte Alarme umfassen.

6. Verfahren nach einem der vorhergehenden Ansprüche, **dadurch gekennzeichnet, dass** die vom Steuergerät (1) an die Zentrale (9) übertragenen Daten im Steuergerat (1) mittels GPS (Global Positioning System) ermittelte Daten über einen momentanen Standort des Steuergerätes (1) umfassen.

7. Verfahren nach einem der vorhergehenden Ansprüche, **dadurch gekennzeichnet, dass** die vom Steuergerät (1) an die Zentrale (9) übertragenen Daten Patientendaten, insbesondere Gewicht, Größe, Body-Mass-Index, umfassen.

8. Verfahren nach einem der vorhergehenden Ansprüche, **dadurch gekennzeichnet, dass** zu Zwecken einer Fernwartung Daten von der Zentrale (9) an das Steuergerät (1) übertragen werden.

9. Verfahren nach einem der vorhergehenden Ansprüche, **dadurch gekennzeichnet, dass** die von der Zentrale (9) an das Steuergerät (1) übertragenen Daten mindestens eine der folgenden Daten umfassen: Software-Aktualisierungen; Patientenparameter, insbesondere Größe und/oder Gewicht; Regelparameter, insbesondere Solldruckwerte; Steuerbefehle, insbesondere Befehle zum Löschen und/oder Zurücksetzen von auf dem Steuergerät (1) gespeicherten Daten .

10. Verfahren nach Anspruch 8 oder 9, **dadurch gekennzeichnet, dass** die von der Zentrale (9) an das Steuergerät (1) übertragenen Daten eine Befehlsfolge zur Durchführung eines Funktionstests an dem Therapiegerät umfassen.

11. Verfahren nach Anspruch 10, **dadurch gekennzeichnet, dass** der Funktionstest eine Leckageprüfung des Therapiegerätes ist, umfassend die Schritte:
- Bestimmen der Pumpenlaufzeit und der Betriebszeit während eines vorbestimmten Zeitintervalls und/oder
- Bestimmen der Anzahl der Ventilschaltungen während eines vorbestimmten Zeitintervalls;
- Feststellen, ob eine Leckage vorliegt, durch Vergleich mit Erfahrungswerten.

12. Verfahren nach Anspruch 11, **dadurch gekennzeichnet, dass** das Feststellen durch eine in der Zentrale (9) implementierte Automatik erfolgt.

13. Medizinisches Therapiegerät mit einem elektronischen Steuergerat (1), mindestens einer elektrischen Pumpe, mindestens einem Ventil und mindestens einem Drucksensor, insbesondere Anti-Dekubitus-Auflage und/oder System zur Unterdruckbehandlung von Wunden, wobei Daten zwischen dem Steuergerät (1) und einer Zentrale (9) mittels bidirektionaler Datenfernübertragung mit einer seriellen Schnittstelle (2), vorzugsweise RS 232 und/oder USB-Schnittstelle, und/oder einer Blue Tooth Schnittstelle und/oder einer W-LAN Schnittstelle zur Verbindung mit dem Internet über ein Modem austauschbar sind, **dadurch gekennzeichnet ,dass** zu Zwecken einer statistischen Auswertung Grunddaten und Betriebsdaten des Therapiegerätes vom Steuergerät (1) an die Zentrale (9) übertragbar sind.

14. Medizinisches Therapiegerät nach Anspruch 13, **dadurch gekennzeichnet, dass** es zur Verschlüsselung der zu übertragenden Daten ausgebildet Ist.

15. Medizinisches Therapiegerät nach Anspruch 13 oder 14, **dadurch gekennzeichnet, dass** es zur Durchführung eines Verfahrens nach einem der Ansprüche 1 bis 12 ausgestaltet ist.

## Claims

1. Method for operating a medical therapy device provided with an electronic control device (1), at least one electrical pump, at least one valve and at least one pressure sensor, in particular an anti-decubitus dressing and/or a system for the negative pressure treatment of wounds, data being interchanged between the control device (1) and a control centre (9) by means of bidirectional remote data transmission, **characterized in that** basic data and operating data relating to the therapy device are transmitted from the control device (1) to the control centre (9) for the purposes of statistical evaluation.

2. Method according to Claim 1, **characterized in that** the data transmitted from the control device (1) to the control centre (9) comprise at least one of the following basic data items:
serial number of the control device (1);
control device basic settings, in particular language and/or unit system.

3. Method according to Claim 1 or 2, **characterized in that** the data transmitted from the control device (1) to the control centre (9) comprise at least one of the following operating data items: operating time; pump running time; number of valve switching operations; desired and actual pressure values; supply voltage of the control device (1); operating state of the pump.

4. Method according to Claim 1 or 2, **characterized in that** the data transmitted from the control device (1) to the control centre (9) are provided with a time stamp.

5. Method according to one of the preceding claims, **characterized in that** the data transmitted from the control device (1) to the control centre (9) comprise alarms produced by the control device (1).

6. Method according to one of the preceding claims, **characterized in that** the data transmitted from the control device (1) to the control centre (9) comprise data relating to a current location of the control device (1) which are determined in the control device (1) by means of GPS (Global Positioning System).

7. Method according to one of the preceding claims, **characterized in that** the data transmitted from the control device (1) to the control centre (9) comprise patient data, in particular weight, height, body mass index.

8. Method according to one of the preceding claims, **characterized in that** data are transmitted from the control centre (9) to the control device (1) for the purposes of remote maintenance.

9. Method according to one of the preceding claims, **characterized in that** the data transmitted from the control centre (9) to the control device (1) comprise at least one of the following data items: software updates; patient parameters, in particular height and/or weight; control parameters, in particular desired pressure values; control commands, in particular commands for deleting and/or resetting data stored on the control device (1).

10. Method according to Claim 8 or 9, **characterized in that** the data transmitted from the control centre (9) to the control device (1) comprise a sequence of commands for carrying out a function test on the therapy device.

11. Method according to Claim 10, **characterized in that** the function test is a leak test of the therapy device, comprising the steps of:
- determining the pump running time and the operating time during a predetermined interval of time, and/or
- determining the number of valve switching operations during a predetermined interval of time;
- determining whether there is a leak by means of comparison with empirical values.

12. Method according to Claim 11, **characterized in that** the determination is carried out by means of an automatic system implemented in the control centre (9).

13. Medical therapy device having an electronic control device (1), at least one electrical pump, at least one valve and at least one pressure sensor, in particular an anti-decubitus dressing and/or a system for the negative pressure treatment of wounds, data being able to be interchanged between the control device (1) and a control centre (9) by means of bidirectional remote data transmission using a serial interface (2), preferably an RS 232 and/or USB interface, and/or a Bluetooth interface and/or a WLAN interface for connection to the Internet via a modem, **characterized in that** basic data and operating data relating to the therapy device can be transmitted from the control device (1) to the control centre (9) for the purposes of statistical evaluation.

14. Medical therapy device according to Claim 13, **characterized in that** it is designed to encrypt the data to be transmitted.

15. Medical therapy device according to Claim 13 or 14, **characterized in that** it is designed to carry out a method according to one of Claims 1 to 12.

## Revendications

1. Procédé de fonctionnement d'un appareil médical thérapeutique comprenant un appareil de commande électronique (1), au moins une pompe électrique, au moins une soupape et au moins un capteur de pression, en particulier un support anti-escarres et/ou un système pour le traitement de plaies par dépression, des données étant échangées entre l'appareil de commande (1) et une centrale (9) au moyen d'un transfert bidirectionnel de données à distance, **caractérisé en ce qu'**aux fins d'une analyse statistique, des données de base et des données fonctionnelles de l'appareil thérapeutique sont transmises de l'appareil de commande (1) à la centrale (9).

2. Procédé selon la revendication 1, **caractérisé en ce que** les données transmises de l'appareil de commande (1) à la centrale (9) comprennent au moins l'une des données de base suivantes :
numéro de série de l'appareil de commande (1) ;
ajustements de base de l'appareil de commande, en particulier langue et/ou système d'unités.

3. Procédé selon la revendication 1 ou 2, **caractérisé en ce que** les données transférées de l'appareil de commande (1) à la centrale (9) comprennent au moins l'une des données fonctionnelles suivantes : durée de fonctionnement ; durée de fonctionnement de la pompe ; nombre de commutations de la soupape ; valeur de pression de consigne et instantanée ; tension d'alimentation de l'appareil de commande (1) ; état de fonctionnement de la pompe.

4. Procédé selon la revendication 1 ou 2, **caractérisé en ce que** les données transmises de l'appareil de commande (1) à la centrale (9) sont pourvues d'un horodatage.

5. Procédé selon l'une quelconque des revendications précédentes, **caractérisé en ce que** les données transmises de l'appareil de commande (1) à la centrale (9) comprennent des alarmes générées par l'appareil de commande (1).

6. Procédé selon l'une quelconque des revendications précédentes, **caractérisé en ce que** les données transmises de l'appareil de commande (1) à la centrale (9) comprennent des données déterminées par GPS (Global Positioning System) dans l'appareil de commande (1) par le biais d'une localisation instantanée de l'appareil de commande (1).

7. Procédé selon l'une quelconque des revendications précédentes, **caractérisé en ce que** les données transmises de l'appareil de commande (1) à la centrale (9) comprennent des données relatives au patient, en particulier son poids, sa taille, son indice de masse corporelle.

8. Procédé selon l'une quelconque des revendications précédentes, **caractérisé en ce qu'**aux fins d'un entretien à distance, des données sont transmises de la centrale (9) à l'appareil de commande (1).

9. Procédé selon l'une quelconque des revendications précédentes, **caractérisé en ce que** les données transmises de la centrale (9) à l'appareil de commande (1) comprennent au moins l'une des données suivantes : mises à jour de logiciels, paramètres du patient, en particulier sa taille et/ou son poids ; paramètres de régulation, en particulier valeurs de pression de consigne ; ordres de commande, en particulier ordres d'effacement et/ou de remise à zéro de données mémorisées sur l'appareil de commande (1).

10. Procédé selon la revendication 8 ou 9, **caractérisé en ce que** les données transmises de la centrale (9) à l'appareil de commande (1) comprennent une série d'ordres pour effectuer un test de fonctionnement de l'appareil thérapeutique.

11. Procédé selon la revendication 10, **caractérisé en ce que** le test de fonctionnement est un contrôle de fuite de l'appareil thérapeutique, comprenant les étapes suivantes :
- détermination de la durée de fonctionnement de la pompe et du temps de fonctionnement pendant un intervalle de temps prédéterminé et/ou
- détermination du nombre de commutations de la soupape pendant un intervalle de temps prédéterminé ;
- établissement de la présence ou non d'une fuite, par comparaison avec des valeurs expérimentales.

12. Procédé selon la revendication 11, **caractérisé en ce que** l'établissement s'effectue par un automatisme mis en oeuvre dans la centrale (9).

13. Appareil médical thérapeutique comprenant un appareil de commande électronique (1), au moins une pompe électrique, au moins une soupape et au moins un capteur de pression, en particulier un support anti-escarres et/ou un système pour le traitement de plaies par dépression, des données pouvant être échangées entre l'appareil de commande (1) et une centrale (9) au moyen d'un transfert bidirectionnel de données à distance avec une interface série (2), de préférence une interface RS 232 et/ou USB, et/ou une interface Bluetooth et/ou une interface W-LAN, pour la connexion à l'Internet par le biais d'un modem, **caractérisé en ce qu'**aux fins d'une analyse statistique, des données de base et des données fonctionnelles de l'appareil thérapeutique peuvent être transmises de l'appareil de commande (1) à la centrale (9).

14. Appareil médical thérapeutique selon la revendication 13, **caractérisé en ce qu'**il est réalisé pour coder les données à transmettre.

15. Appareil médical thérapeutique selon la revendication 13 ou 14, **caractérisé en ce qu'**il est configuré pour mettre en oeuvre un procédé selon l'une quelconque des revendications 1 à 12.
